# EUROPÄISCHE PATENTSCHRIFT

(11) **EP 2 617 412 B1**
(45) Veröffentlichungstag und Bekanntmachung des Hinweises auf die Patenterteilung: **30.12.2015**
(21) Anmeldenummer: 12006743.4
(22) Anmeldetag: 27.09.2012
(51) Int. Cl.: A61P 29/00, A61K 36/185, A61K 36/09, A61K 36/73, A61K 36/48, A61K 36/68, A61K 9/00, A61K 9/20

(54) **Zusammensetzung für die topische Behandlung**
Composition for topical treatment
Composition pour le traitement topique

(30) Priorität: 20.01.2012 DE 102012000976
(43) Veröffentlichungstag der Anmeldung: 24.07.2013
(73) Patentinhaber: Maria Clementine Martin Klosterfrau Vertriebsgesellschaft mbH, 50670 Köln (DE)
(72) Erfinder: Greve, Harald, 40545 Düsseldorf (DE); Vestweber, Anna-Maria, 51399 Burscheid (DE)
(74) Vertreter: Strehlke, Ingo Kurt

(56) Entgegenhaltungen:
- EP-A1- 1 639 998
- DE-U1-202004 016 077
- DE-U1-202004 016 078
- MESSADI D V ET AL: "Aphthous ulcers", DERMATOLOGIC THERAPY 2010 BLACKWELL PUBLISHING INC. USA, Bd. 23, Nr. 3, Mai 2010 (2010-05), Seiten 281-290, XP009164881, ISSN: 1396-0296
- GRUBER I ET AL: "[Local anesthetic effects on the gingiva of two mucosal preparations].", DIE QUINTESSENZ OCT 1990, Bd. 41, Nr. 10, Oktober 1990 (1990-10), Seiten 1677-1682, XP009164884, ISSN: 0033-6580

## Beschreibung

Die vorliegende Erfindung betrifft das medizinische Gebiet der Behandlung entzündlicher Erkrankungen des Mund-/Rachenraumes.

Insbesondere betrifft die vorliegende Erfindung eine pharmazeutische Zusammensetzung zur Verwendung bei der topischen Behandlung entzündlicher Erkrankungen des Mund- und Rachenraumes. Darüber hinaus betrifft die vorliegende Erfindung die Verwendung der erfindungsgemäßen Zusammensetzung für die vorgenannten Anwendungen.

Entzündliche Erkrankungen des Mund- und Rachenraums, d. h. Entzündungen im Bereich der Mundhöhle und des Hals-/Rachenraums, treten oftmals als Begleiterscheinung von Erkältungserkrankungen und grippalen Infekten, aber auch als eigenständige Erkrankungen auf. Solche entzündlichen Prozesse des Mund- und Rachenraums sind oftmals mit einer für den betroffenen Patienten unangenehmen Schmerzsymptomatik verbunden. Nicht beschränkende Beispiele für derartige entzündliche Erkrankungen des Hals-/Rachenraums sind Halsentzündungen, insbesondere Angina, Entzündungen des Kehlkopfs (Laryngitis), Entzündungen der Rachenschleimhaut (Pharyngitis) und Entzündungen der Rachenmandeln (Tonsillitis). Solche Erkrankungen des Hals-/Rachenraums sind oftmals von schmerzhaften Schluckbeschwerden begleitet. Auch Entzündungen im Bereich der Mundhöhle, wie z. B. Stomatitis, Gingivitis, Mundschleimhautläsionen und dergleichen, sind oftmals von Schmerzen begleitet.

Für weitere diesbezügliche Einzelheiten zu den vorgenannten Erkrankungen kann beispielsweise auf Roche-Lexikon Medizin, 3. Auflage, 1993, Urban & Schwarzenberg, München/WienBaltimore, sowie auf Pschyrembel, Medizinisches Wörterbuch, 257. Auflage, 1993, Nikol Verlagsgesellschaft mbH, Hamburg, verwiesen werden. Der Begriff "entzündliche Erkrankungen des Mund- und Rachenraums" ist somit sehr weit zu verstehen und umfasst sämtliche entzündlichen Erkrankungen, welche im Bereich der Mundhöhle, des Rachens und des Halsraums auftreten können.

Die Behandlung entzündlicher Erkrankungen des Mund- und Rachenraums erfolgt - in Abhängigkeit von der Schwere des Krankheitsbildes - im Allgemeinen durch topische und gegebenenfalls zusätzliche systemische Therapie in schwerwiegenderen Fällen.

Während in schwereren Fällen systemisch Antibiotika verabreicht werden, kommen unterstützend und in leichteren Fällen unter Umständen alleinig zur topischen, symptomatischen Behandlung oftmals Antiseptika und entzündungshemmende Stoffe (so z. B. Antiphlogistika, antiinflammatorisch wirkende Mittel, Lokalantibiotika etc.) zum Einsatz, welche beispielsweise in Form von Rachenspülungen, Sprays oder Lutschtabletten verabreicht werden können.

Als Wirkstoff zur topischen Behandlung von entzündlichen Prozessen des Mund- und Rachenraums hat sich insbesondere 1-Hexadecylpyridiniumchlorid (internationaler Freiname: "Cetylpyridiniumchlorid" (CPC)) bewährt. Hierbei handelt es sich um eine quaternäre Ammoniumverbindung mit bakterizider und fungizider Wirkung, welche unter anderem in Lutschtabletten zur Anwendung kommt. Nachteilig bei diesem Wirkstoff ist die Tatsache, dass bei hoher Dosierung und übermäßigem Verzehr Magen-Darm-Beschwerden,
Atemnot sowie eine vermehrte Methämoglobinbildung, insbesondere bei Kindern, auftreten kann.

Des Weiteren hat sich als topisches Antiseptikum bzw. Desinfektionsmittel für die Mund-, Hals- und Rachenschleimhaut auch 1,3-Bis(2-ethylhexyl)-hexahydro-5-methyl-5-pyridinamin (internationaler Freiname: "Hexetidin") bewährt, welches beispielsweise als Spray oder in Form von Spül- bzw. Gurgellösungen appliziert werden kann. Bei längerer Anwendung und starker Dosierung kann es auch hier zu Magen-Darm-Beschwerden und darüber hinaus zu Geschmacksirritationen kommen.

Um darüber hinaus eine Schmerzlinderung im entzündeten Mund-, Hals- und Rachenbereich zu erzielen, werden teilweise Lokalanästhetika, wie beispielsweise Benzocain, Procain oder Lidocain, eingesetzt. Zwar kann durch den Einsatz der vorgenannten Lokalanästhetika ein Rückgang der Schmerzen erzielt werden, allerdings ist das Ausmaß der anästhetischen Wirkung mitunter nur schwer zu kontrollieren. Unter Umständen kann es insbesondere unmittelbar nach Applikation des Lokalanästhetikums zu einer unerwünscht starken Betäubung des Gewebes kommen. Weiterhin führen Lokalanästhetika der vorgenannten Art, insbesondere Benzocain, unter Umständen zu Unverträglichkeiten bei Anwendung auf der empfindlichen Mund-, Hals- und Rachenschleimhaut.

Des Weiteren kommen Wirkstoffe natürlichen Ursprungs zum Einsatz, so z. B. sogenannte Schleimdrogen oder deren Extrakte, wie z. B. Isländisches Moos (*Lichen islandicus*). Derartige Präparate bieten aber nicht immer den gewünschten Therapieerfolg. Insbesondere berücksichtigen diese Präparate im Allgemeinen nicht die mit den entzündlichen Erkrankungen des Mund- und Rachenraums einhergehende Schmerzsymptomatik.

Die DE 20 2004 016 078 U1, die EP 1 639 998 A1 und die DE 20 2004 016 077 U1 betreffen Zusammensetzungen in fester Dosierungsform, welche zur Behandlung von entzündlichen Erkrankungen des Mund- und Rachenraums mindestens eine Schleimdroge, insbesondere Isländisches Moos bzw. Eibisch, und mindestens ein Lokalanästhetikum enthalten.

Die wissenschaftliche Publikation gemäß Messadi et al.: "Aphthous ulcers", Dermatologic Therapy 2010, Blackwell Publishing Inc., USA*,* betrifft den Einsatz von Polidocanol in Form einer Zahncreme zur Behandlung von Aphten.

Weiterhin betrifft die Publikation gemäß Gruber et al: "Local anesthetic effects on the gingiva of two mucosal preparations*" Die Quintessenz,* eine medizinische Studie zur Untersuchung des Wirkeffekts verschiedener Lokalanästhetika, insbesondere von Lidocain und Polidocanol, bei Anwendung auf der Mundschleimhaut.

Der vorliegenden Erfindung liegt daher die Aufgabe zugrunde, eine Zusammensetzung, insbesondere eine pharmazeutische Zusammensetzung, bereitzustellen, welche die zuvor geschilderten Nachteile des Standes der Technik zumindest weitgehend vermeidet oder aber wenigstens abschwächt. Insbesondere liegt der vorliegenden Erfindung die Aufgabe zugrunde, eine Zusammensetzung bereitzustellen, welche sich zur effizienten topischen Behandlung von entzündlichen Erkrankungen des Mund- und Rachenraums (d. h. also von entzündlichen Erkrankungen des Hals-/Rachenraumes und der Mundhöhle) eignet.

Weiterhin liegt der vorliegenden Erfindung die Aufgabe zugrunde, eine Zusammensetzung bereitzustellen, welche einerseits in effizienter Weise Entzündungen und Reizungen der Haut bzw. Schleimhaut im Mund- bzw. Rachenbereich lindert und andererseits eine gezielte und kontrollierte Behandlung von Schmerzzuständen im Mund- bzw. Rachenbereich erlaubt, sich gleichzeitig jedoch auch durch eine hervorragende Verträglichkeit auszeichnet.

Zur Lösung der zuvor geschilderten Aufgabe schlägt die vorliegende Erfindung eine Zusammensetzung nach Anspruch 1 vor; weitere vorteilhafte Ausgestaltungen sind Gegenstand der diesbezüglichen abhängigen Ansprüche.

Weiterer Gegenstand der vorliegenden Erfindung ist zudem die Verwendung der Zusammensetzung nach der Erfindung gemäß den diesbezüglichen Ansprüchen.

Es versteht sich von selbst, dass im Folgenden besondere Ausgestaltungen, Ausführungsformen oder dergleichen, welche nur im Zusammenhang mit einem Erfindungsaspekt beschrieben sind, auch in Bezug auf die anderen Erfindungsaspekte entsprechend gelten, ohne dass dies einer ausdrücklichen Erwähnung bedarf.

Weiterhin ist bei allen nachstehend genannten relativen bzw. prozentualen, insbesondere gewichtsbezogenen Mengenangaben zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen stets 100 % resultieren. Dies versteht sich für den Fachmann aber von selbst.

Im Übrigen gilt, dass der Fachmann anwendungsbezogen oder einzelfallbedingt von den nachfolgend aufgeführten Zahlen-, Bereichs- oder Mengenangaben abweichen kann, ohne dass er den Rahmen der vorliegenden Erfindung verlässt.

Zudem gilt, dass alle im Folgenden genannten Parameterangaben oder dergleichen grundsätzlich mit genormten oder explizit angegebenen Bestimmungsverfahren oder aber mit dem Fachmann an sich geläufigen Bestimmungsmethoden bestimmt bzw. ermittelt werden können.

Gegenstand der vorliegenden Erfindung ist somit - gemäß einem **ersten** Erfindungsaspekt - eine pharmazeutische Zusammensetzung in Form einer festen Dosierung zur Verwendung bei der topischen Behandlung von entzündlichen Erkrankungen des Mund- und Rachenraumes, wobei die feste Dosierungsform eine Tablette, Dragee, Pille oder Hartkaramelle ist und wobei die pharmazeutische Zusammensetzung in Kombination
(a) in einer relativen Menge im Bereich von 0,1 bis 25 Gew.-%, bezogen auf die pharmazeutische Zusammensetzung, mindestens eine Schleimdroge und/oder deren Extrakt; und
(b) in einer relativen Menge im Bereich von 0,1 bis 9 Gew.-%, bezogen auf die pharmazeutische Zusammensetzung, Polidocanol als Lokalanästhetikum;
aufweist,
wobei die Schleimdroge ausgewählt ist aus Isländischem Moos (*Lichen islandicus*)*,* Eibisch (*Althaea officinalis L.*), Spitzwegerich (*Plantago lanceolata L.*)*,* Malve (*Malva sylvestris L.* und *M. neglecta WALLR.*)*,* Bockshornklee (*Trigonella foenum-graecum L.*), Salep und Quitte (*Cydonia oblonga MILL.*) und deren Kombinationen und
wobei die Zusammensetzung (a) die mindestens eine Schleimdroge und/oder deren Extrakt und (b) Polidocanol in einem gewichtsbezogenen Verhältnis von [(a) : (b)] im Bereich von 1 : 5 bis 5 : 1 enthält.

Die Anmelderin hat nunmehr in überraschender Weise herausgefunden, dass eine pharmazeutische Zusammensetzung in Form einer festen Dosierung, welche als Wirkstoffe mindestens eine Schleimdroge bzw. deren Extrakte einerseits und Polidecanol als Lokalanästhetikum andererseits enthält, eine besonders effiziente Reizlinderung bzw. Entzündungshemmung sowie eine gezielte Schmerzlinderung im Rahmen der prophylaktischen und/oder therapeutischen Behandlung von entzündlichen Erkrankungen des Mund- und Rachenraumes ermöglicht. In diesem Zusammenhang hat sich insbesondere gezeigt, dass die Wirkung der erfindungsgemäßen Kombination über die Wirkung der Einzelwirkstoffe in signifikanter Weise hinausgeht und somit synergistisch ist. Darüber hinaus ist die pharmazeutische Zusammensetzung zumindest im Wesentlichen frei von Nebenwirkungen.

Die Begriffe "pharmazeutische Zusammensetzung", "pharmazeutische Zubereitung" oder dergleichen, wie sie im Rahmen der vorliegenden Erfindung verwendet werden, sind sehr umfassend zu verstehen und bezeichnen nicht nur pharmazeutische Präparate bzw. Pharmazeutika, sondern auch sogenannte Medizinprodukte, homöopathische Mittel, Nahrungsergänzungsmittel oder dergleichen.

Die im Rahmen der vorliegenden Erfindung eingesetzten Schleimdrogen werden üblicherweise wegen ihrer antiphlogistischen Eigenschaften äußerlich zur Behandlung von Furunkeln, Geschwüren, Drüsenschwellungen und Entzündungen des Rachenraumes, innerlich auch als Laxans, Antidiarrhoikum und zur Behandlung von Magen- und Darmentzündungen verwendet.

Chemisch gesehen handelt es sich bei Schleimen um Heteropolysaccharide mit Molekulargewichten von ca. 50.000 bis 2.000.000 Da, die durch Extraktion mit heißem oder kaltem Wasser aus der Droge gewonnen werden. Die hochviskosen Lösungen sind im Gegensatz zu den Gummen nicht klebrig. Man unterscheidet saure und neutrale Schleime. Je nach ihrer Zuckerzusammensetzung lassen sie sich in Glucomannane oder Mannane, in Galactomannane, Xylane oder Rhamnogalacturonane einteilen. Nach ihrer Lokalisierung in der Pflanze kann man Vakuolenschleime und Membranschleime unterscheiden. Als Prototyp für einen sauren Pflanzenschleim kann der Schleim der Eibischwurzel angesehen werden. Er enthält L-Rhamnose, D-Galactose, D-Galacturonsäure und D-Glucuronsäure im molaren Verhältnis von ungefähr 3 : 2 : 3 : 3. Der am einfachsten gebaute Teil des Polysaccharids besteht aus sich wiederholenden Undecasaccharid-Untereinheiten. Für weitergehende Informationen zum Begriff der Schleimdrogen bzw. Schleime kann verwiesen werden auf Römpp Chemielexikon, 10. Auflage, Georg Thieme Verlag, Stuttgart / New York, Band 5, Stichwort "Schleim", Seite 3969, sowie die darin referierte Literatur, deren diesbezüglicher Inhalt hiermit vollumfänglich durch Bezugnahme eingeschlossen ist.

Bei dem im Rahmen der vorliegenden Erfindung verwendeten Polidocanol handelt es sich gemäß der Definition des Europäischen Arzneibuchs 6.0 um ein Gemisch von Ethern verschiedener Macrogole mit Fettalkoholen, hauptsächlich Laurylalkohol. Synonym wird Polidocanol auch als Hydroxypolyethoxydodecan, Macrogollaurylether oder Lauromacrogol 400 bezeichnet. Aufgrund des lipophilen Dodecylrests und der hydrophilen Etherkette lässt sich Polidocanol gut mit Wasser mischen. Darüber hinaus besitzt Polidocanol oberflächenaktive Eigenschaften. Im Stand der Technik wird Polidocanol im Gebiet der Medizin insbesondere zur Verödung von Hämorrhoiden oder Besenreisern in das betroffene Gewebe injiziert; darüber hinaus kann Polidocanol im Stand der Technik in Hautsalben zur Schmerz- und Juckreizlinderung der äußeren Haut (Lederhaut bzw. Dermis) verwendet werden. Im Rahmen der vorliegenden Erfindung kann überraschenderweise erstmals eine Applikation von Polidocanol auf der empfindlichen Schleimhaut realisiert werden, insbesondere im Zusammenwirken mit der Schleimdroge wird in vollkommen überraschender Weise keinerlei Reizung der Schleimhäute beobachtet. Für weitergehende Einzelheiten zum Wirkstoff "Polidocanol" kann verwiesen werden auf Römpp, Chemielexikon, 10. Auflage, Georg Thieme Verlag, Stuttgart / New York, Band 5, Seite 3403, Stichwort: "Polidocanol", sowie auf die darin referierte Literatur, deren diesbezüglicher Inhalt hiermit vollumfänglich durch Bezugnahme eingeschlossen ist.

Die Anmelderin hat nämlich überraschenderweise gefunden, dass sich durch die synergistische Kombination von mindestens einer Schleimdroge einerseits sowie Polidocanol als Lokalanästhetikum andererseits in festen Dosierungsformen, wie z.B. Lutschtabletten oder Hartkaramellen, die Verträglichkeit gegenüber herkömmlichen Zusammensetzungen zur Behandlung von entzündlichen Erkrankungen des Mund-/Rachenraumes signifikant steigern lässt, die Zusammensetzung aber dennoch eine hervorragende lokalanästhetische Wirkung und darüber hinaus ausgezeichnete entzündungshemmende Eigenschaften besitzt.

Die vorliegende Erfindung weist darüber hinaus zahlreiche weitere Vorteile und Besonderheiten auf, welche sie gegenüber dem Stand der Technik auszeichnen und nachfolgend ausführlich geschildert sind.

In vollkommen überraschender Weise hat die Anmelderin im Rahmen ihrer aufwendigen und zeitintensiven Forschungsarbeit herausgefunden, dass der Wirkstoff Polidocanol auch bei Anwendung auf der Schleimhaut, insbesondere im Mund-/Rachenraum, eine hervorragende Effektivität in Bezug auf die Schmerzlinderung besitzt. Im Rahmen der vorliegenden Erfindung wird dies - ohne sich hierbei auf diese Theorie beschränken zu wollen - insbesondere auf die amphiphilen Eigenschaften des Polidocanols zurückgeführt, da einerseits eine gute Haftung an der Schleimhaut im Mund-/Rachenraum sowie andererseits eine erhöhte Bindungskapazität für die Rezeptoren der Nervenzellen gewährleistet wird.

Was die lokalanästhetische Wirkung des Polidocanols im Speziellen anbelangt, so lässt sich diese bei Einsatz von Polidocanol in definierten Mengen sowie in einem definierten gewichtsbezogenen Mengenverhältnis zu der Schleimdroge sehr zielgerichtet steuern und an die erwünschte Wirkintensität anpassen. Insbesondere ist es möglich, über die Menge bzw. das Mengenverhältnis zur Schleimdroge die Wirkungsstärke derart einzustellen, dass eine äußerst lang anhaltende und nachhaltige Schmerzlinderung erzielt wird, die Anfangsintensität der Schmerzlinderung aber dennoch von den Patienten nicht als unangenehm stark empfunden wird. Darüber hinaus zeichnet sich Polidocanol im Rahmen der erfindungsgemäßen Anwendung gegenüber den im Stand der Technik üblicherweise verwendeten Lokalanästhetika, wie beispielsweise Benzocain, durch eine signifikant verbesserte Verträglichkeit aus. Auch bei häufiger Anwendung treten zumindest im Wesentlichen keine Irritationen oder ähnliche Unverträglichkeitsreaktionen an der Schleimhaut auf. Erfindungsgemäß wird davon ausgegangen - ohne sich hierbei auf diese Theorie beschränken zu wollen - dass die äußerst gute Verträglichkeit von Polidocanol ebenfalls auf die Molekülstruktur, insbesondere dessen amphiphile Eigenschaften, zurückzuführen ist, da ein tiefes Eindringen der Einzelmoleküle in die Schleimhaut nicht möglich ist. Mit anderen Worten führt der geringe Invasivitätsgrad von Polidocanol zu dessen nebenwirkungsfreier Anwendung.

Im Zusammenhang mit den zuvor beschriebene Vorteilen und Besonderheiten wird bereits an dieser Stelle auf die von der Anmelderin durchgeführten Wirksamkeitsstudien verwiesen, welche die vorgenannten Effekte in eindrucksvoller Weise belegen und nachfolgend noch detailliert beschrieben sind.

Die erfindungsgemäße Zusammensetzung kann in vielfältiger Art und Weise ausgestaltet sein. Bevorzugte Ausführungsformen sind im Folgenden zum besseren Verständnis beschrieben.

Gemäß einer ersten Ausführungsform der vorliegenden Erfindung kann es vorgesehen sein, dass die Zusammensetzung die mindestens eine Schleimdroge in Form der Droge, insbesondere in Form zerkleinerter oder pulverisierter Pflanzenteile oder Pflanzenbestandteile, enthält.

Gemäß einer zweiten und besonders bevorzugten Ausführungsform kann es insbesondere vorgesehen sein, dass die Zusammensetzung die mindestens eine Schleimdroge in Form eines Extraktes, insbesondere eines Trockenextraktes, enthält. Insbesondere im Zusammenhang mit der Linderung der Entzündungssymptomatik bei Erkrankungen des Hals- und Mundraumes haben sich Trockenextrakte in ihrer Wirksamkeit - insbesondere aufgrund der hohen Wirkstoffkonzentration - als besonders effektiv erwiesen. Dabei ist es besonders bevorzugt, wenn der Extrakt ausgehend von einem wässrigen, alkoholischen oder wässrig-alkoholischen Extrakt, bevorzugt einem wässrigen Extrakt, erhältlich ist.

Darüber hinaus lässt sich die Wirksamkeit der erfindungsgemäßen Zusammensetzung noch weitergehend steigern, wenn die Zusammensetzung die mindestens eine Schleimdroge in Form eines Extraktes, vorzugsweise eines Trockenextraktes, mit einem Droge/Extrakt-Verhältnis im Bereich von 0,2 : 1 bis 20 : 1, insbesondere im Bereich von 1 : 1 bis 15 : 1, vorzugsweise im Bereich von 5 : 1 bis 10 : 1, enthält.

Das Droge/Extrakt-Verhältnis (DEV) gibt an, aus welcher gewichtsbezogenen Menge an eingesetzter Droge, z. B. Isländisches Moos, welche gewichtsbezogene Menge Extrakt gewonnen wurde. Ein Droge/Extrakt-Verhältnis beispielsweise von 6 : 1 bedeutet, dass aus sechs Gewichtsteilen Droge ein Gewichtsteil Extrakt gewonnen wurde. Das Droge/Extrakt-Verhältnis gibt also an, wie viele Gewichtsteile einer Arzneidroge für die Herstellung des gewichtsbezogenen Extraktäquivalents benötigt werden.

Die Verwendung von Extrakten mit definiertem Droge/Extrakt-Verhältnis ist dahingehend relevant, dass die Qualität des Extraktes einen entscheidenden Einfluss auf die Gesamtqualität der pharmazeutischen Zubereitung hat. Ziel ist es dabei, den Extrakt hinsichtlich der Wirkstoffkonzentration zu standardisieren, so dass auch die finale pharmazeutische Zubereitung qualitativ und quantitativ mit konstant guten Werten bezüglich der Wirk- und Inhaltsstoffe bereitgestellt werden kann.

Was die eingesetzte Menge der Schleimdroge in der Gesamtzusammensetzung anbelangt, so ist diese variabel. Erfindungsgemäß ist es vorgesehen, dass die Zusammensetzung die mindestens eine Schleimdroge und/oder deren Extrakt in einer relativen Menge im Bereich von 0,1 bis 25 Gew.-%, insbesondere im Bereich von 1 bis 10 Gew.-%, bezogen auf die pharmazeutische Zusammensetzung, enthält.

Gleichermaßen kann es im Rahmen der vorliegenden Erfindung vorgesehen sein, dass die erfindungsgemäße Zusammensetzung die mindestens eine Schleimdroge und/oder deren Extrakt in einer absoluten Menge im Bereich von 1 bis 500 mg, insbesondere im Bereich von 10 bis 250 mg, vorzugsweise im Bereich von 20 bis 200 mg, bevorzugt im Bereich von 30 bis 100 mg, bezogen auf eine Applikationseinheit, enthält.

Unter einer Applikationseinheit bzw. einer Applikationsmenge ist insbesondere die Menge an erfindungsgemäßer Zusammensetzung zu verstehen, welche im Rahmen einer einzelnen Behandlung(s)einheit, z. B. bei einer einzelnen Lutschtablette bzw. bei Verabreichung einer einzelnen Lutschtablette, verwendet wird.

Für den erfindungsgemäßen Einsatz in der erfindungsgemäßen festen Dosiereinheit können verschiedene Schleimdrogen - sowohl einzeln als auch in Kombination - eingesetzt werden. Erfindungsgemäß ist die mindestens eine Schleimdroge ausgewählt aus Isländischem Moos (*Lichen islandicus*), Eibisch (*Althaea officinalis L.*), Spitzwegerich (*Plantago lanceolata L.*), Malve (*Malva sylvestris L.* und *M. neglecta WALLR.*), Boxhornklee (*Trigonella foenum-graecum L.*), Salep und Quitte (*Cydonia oblonga MILL.*) und deren Kombinationen, insbesondere aus Isländischem Moos (*Lichen islandicus*) und/oder Eibisch (*Althaea officinalis L.*), vorzugsweise aus Isländischem Moos (*Lichen islandicus*).

Isländisches Moos ist der deutsche Name für *Lichen islandicus* oder *Cetraria islandica* (*Parmeliaceae*), einer Flechte - entgegen dem deutschen Namen kein Moos -, die aus nördlichen Ländern, wie beispielsweise von Island, Norwegen und Schweden, exportiert wird. Isländisches Moos im eigentlichen Sinne besteht aus dem getrockneten Thallus von *Cetraria islandica* sowie dessen Zubereitungen. Die Droge enthält unter anderem Schleimstoffe und Bitterstoffe sowie bitterschmeckende Flechtensäuren. Aus der gepulverten Flechte lösen sich etwa 60 Gew.-% beim Kochen mit stark verdünnter Natriumhydrogencarbonatlösung, und beim Abkühlen der Lösung entsteht eine Gallerte. Der Extrakt besteht aus einem Polysaccharidgemisch von Lichenin und Isolichenin, einer Reihe von bitterschmeckenden Flechtensäuren (Fumarprotocetrar-, Protocetrar- und Cetrarsäure) sowie Protolichesterinsäure, die sich bei der Aufarbeitung in Lichesterinsäure umwandelt, und Usninsäure als antibiotisch wirkendem Flechtenfarbstoff. Als Schleimdroge besitzt Isländisches Moos reizlindernde Eigenschaften, es wirkt ebenso antimikrobiell. Der Bitterstoffgehalt begründet seine Anwendung bei Appetitlosigkeit. Isländisches Moos wird beispielsweise bei Katarrhen und Durchfall, als Bittertonikum, äußerlich bei schlecht heilenden Wunden, bei kosmetischen Präparaten, bei Appetitlosigkeit sowie bei Schleimhautreizungen im Mund- und Rachenraum angewandt. Ein Mund- und Rachendesinfiziens auf Basis von Isländischem Moos wirkt reizlindernd bei trockenem Reizhusten. Das Kaltmazerat und andere bitterschmeckende Zubereitungen der Droge werden zur Behebung der Sub- oder Antazidität eingesetzt. Als Darreichungsformen werden je nach Anwendungsgebiet die zerkleinerte Droge für Aufgüsse sowie andere galenische Zubereitungen bzw. die zerkleinerte Droge vorzugsweise für Kaltmazerate sowie andere bitterschmeckende Zubereitungen zum Einnehmen angewandt. Für weitergehende Einzelheiten zu Isländischem Moos kann beispielsweise auf die folgende Literaturstellen verwiesen werden: Römpp Chemie-Lexikon, 9. Auflage, Band 3, Georg Thieme Verlag, Stuttgart/New York, 1990, Seiten 2055/2056, Stichwort: "Isländisches Moos"; HagerROM 2001, Springer Verlag, Heidelberg, Stichworte: "Cetraria", "Cetraria ericetorum OPIZ", "Cetraria islandica (L.) ACHARIUS" und "Lichen islandicus (Isländisches Moos)"; Monographie "Lichen islandicus (Isländisches Moos)", Bundesanzeiger Nr. 43 vom 2. März 1989; H. Wagner, "Pharmazeutische Biologie - Drogen und ihre Inhaltsstoffe", Gustav Fischer Verlag, Stuttgart/New York, 1985, Seite 281, Stichwort: "Cetrariae Lichen (= Lichen islandicus - Isländisches Moos)".

Der Eibisch (*Althaea officinalis L.*), eingesetzt in Form der Eibischwurzel, -blätter und -blüten, wächst auf salzhaltigen und feuchten Böden Mittel-, Ost- und Südosteuropas. Die im Herbst geernteten, von der holzigen Hauptwurzel, von Wurzelfasern und Rindenschichten befreiten und bei 35 °C getrockneten Wurzelzweige und Nebenwurzeln kommen geschält oder ungeschält in den Handel. Die Braunfärbung der geschälte Droge bedeutet eine Qualitätsminderung; ein nachträgliches "Schönen" mit Sulfitlösung ist unzulässig. Weiterhin kommen die vor oder während der Blüte gesammelten, etwa 10 cm langen, etwa 8 cm breiten, drei- bis fünflappigen, graufilzig behaarten getrockneten Blätter und die im Juli/August gesammelten, fleischfarbenen getrockneten Kronblätter zur Anwendung. Der Eibisch ist eine mehrjährige, etwa 1 m hohe Staude, die generativ oder vegetativ vermehrt wird. Die im Spätherbst geerntete Wurzel enthält bis zu 15 % Schleimstoffe. Im Frühjahr und Sommer liegt der Schleimgehalt bei 5 bis 6 %. Der Schleimgehalt der Blatt- und Blütendroge beträgt 6 bis 9 %. Der Schleim ist in der Wurzel in bestimmten Schleimzellen des Rinden- und Holzparenchyms lokalisiert. Er besteht aus Galacturonorhamnanen, Glucanen und Arabinogalactanen. Wässrige Auszüge aus der Wurzel sollen durch Mazeration bei Zimmertemperatur hergestellt werden, damit die reichlich vorhandene Stärke nicht durch Quellung störend wirkt. Eibischdrogen werden für Teezubereitungen oder zur Herstellung von Sirupus Althaeae verwendet. Das Hauptanwendungsgebiet sind entzündliche Reizzustände des Rachenraums. Äußerlich kommt der Eibisch zu erweichenden Umschlägen, Bädern und Kataplasmen zur Anwendung.

Der Spitzwegerich (*Plantago lanceolata L.*) ist in ganz Europa sowie in Nord- und Mittelasien verbreitet. Die Droge stammt von wild wachsenden Pflanzen und Kulturen vor allem Südosteuropas. Die getrockneten, oliv- bis bräunlichgrün gefärbten, lanzettlichen Blattspreiten mit etwa drei bis sieben parallel verlaufenden Nerven können verwendet werden. Die Herba-Droge besteht aus den getrockneten Blättern, Stengeln und ganzen Blüten. Neben dem Schleim enthält der Spitzwegerich als weitere Inhaltsstoffe Tannine, das Iridoidglykosid Aucubin (1,9 bis 2,4 %), das für die Dunkelfärbung einer nicht sorgfältig getrockneten Droge verantwortlich ist und zur Identitätsprüfung herangezogen wird, ferner das Senföl Sulforaphen. Der Spitzwegerich, insbesondere das Spitzwegerichblätter-Kraut, findet insbesondere Anwendung in Form von Tees und Sirupen bei Entzündungen des Mund- und Rachenraums.

Die Malve (*Malva sylvestris L.* und *M. neglecta WALLR.*)*,* insbesondere eingesetzt in Form von Malvenblüten und -blättern, ist in Europa, Kleinasien, im Mittelmeergebiet und in Vorderindien heimisch. Zur Anwendung kommen die zur Blütezeit gesammelten, rosa-violett gefärbten Blüten bzw. die durch das Trocknen stark eingeschrumpften, gefalteten Blätter, die zumeist in Paketform in den Handel kommen. Die Malve ist ein zwei- bis mehrjähriges Kraut. Der Schleimgehalt von Blüten und Blättern liegt bei 6 bis 8 %. Bei der Hydrolyse liefert der Schleim Glucose, Arabinose, Rhamnose und Galactose. Malvenblüten werden zu Gurgelwässern und Bädern und zusammen mit den Blättern in Teemischungen als Expectorans verwendet.

Der Bockshornklee (*Trigonella foenum-graecum L.*), insbesondere eingesetzt in Form des Bockshornkleesamens, ist im gesamten Mittelmeergebiet, sowie in Osteuropa, Indien und China beheimatet. Der Hauptdrogenimport stammt aus den Kulturen Indiens und Marokkos. Zur Anwendung kommen die braunrötlich gefärbten, vierseitigen und rautenförmigen, etwa 5 mm langen und etwa 3 mm breiten, flachgedrückten, sehr harten Samen, die durch eine Furche charakterisiert sind. Die Droge enthält außer fettem Öl 20 bis 30 % Schleim, Trigonellin, Nicotinsäureamid, Cholin, Bitterstoffe und Saponine. Die Herstellung des Schleims erfolgt aus dem gepulvertem Samen. Das Pulver wird angewendet äußerlich zu Umschlägen bei Furunkeln, Geschwüren und Drüsenschwellungen, innerlich als Expectorans und Roborans.

Salep bzw. Salep Tuber, insbesondere eingesetzt in Form der Salepknolle, ist von verschiedenen Orchideen-Arten abgeleitet, besonders *Orchis mascula L., Orchis morio L., Orchis militaris L., Anacamptis pyramidalis L. RICH.* und *Platanthera bifolia L. RICH.* Zur Anwendung kommen die etwa 4 cm langen bis etwa 3 cm dicken, runzeligen, harten, bräunlichen Knollen, die nach der Ernte von der Korbschicht befreit, mit siedendem Wasser abgebrüht und bei künstlicher Wärme getrocknet werden. Salepschleim, der bis zu 50 % vorwiegend aus Glucomannan bzw. Glucan besteht, wird hauptsächlich in der Kinderheilpraxis als Antidiarrhoikum verwendet.

Die Quitte (*Cydonia oblonga MILL.*) ist im südöstlichen Arabien heimisch. Hautdrogenimporte kommen aus Spanien, Portugal und Persien. Zur Anwendung kommen die reifen, getrockneten, rot-braun bis braun-violett gefärbten, etwas abgeplatteten Samen der Quittenscheinfrucht. Die Samen sind außen zum Teil mit einer eingetrockneten Schleimkruste versehen und oft miteinander verklebt. Sie besitzen einen schwachen Bittermandelgeschmack. In der Epidermis findet sich ca. 22 % Schleim, der zum größten Teil wasserlöslich ist. Die Zuckerkomponenten sind Arabinose, Xylose und Uronsäure, die zum Teil methyliert ist. Die Droge kommt nur unzerkleinert zur Anwendung. Der Quittenschleim wird äußerlich bei Lippen- und Brustwarzenrhagaden, bei Verbrennungen und Dekubitus in Salben- oder Cremeform verwendet.

Erfindungsgemäß besonders gute Ergebnisse werden mit Isländischem Moos und/oder Eibisch, insbesondere mit Isländischem Moos, erzielt.

Was die eingesetzte Menge an Polidocanol in der erfindungsgemäßen Zusammensetzung anbelangt, so ist auch diese variabel. Insbesondere ist es im Rahmen der vorliegenden Erfindung möglich, über die eingesetzte Menge des Polidocanols die Stärke des lokalanästhetischen Effekts gezielt zu steuern. Erfindungsgemäß ist es somit vorgesehen, dass Polidocanol in der erfindungsgemäßen Zusammensetzung in einer relativen Menge im Bereich von 0,1 bis 9 Gew.-%, vorzugsweise im Bereich von 0,5 bis 8 Gew.-%, bevorzugt im Bereich von 1 bis 7 Gew.-%, besonders bevorzugt im Bereich von 2 bis 5 Gew.-%, bezogen auf die pharmazeutische Zusammensetzung, enthalten ist.

Auch kann es vorgesehen sein, dass die Zusammensetzung Polidocanol in einer absoluten Menge im Bereich von 1 bis 500 mg, insbesondere im Bereich von 2 bis 250 mg, vorzugsweise im Bereich von 5 bis 200 mg, bevorzugt im Bereich von 10 bis 150 mg, besonders bevorzugt im Bereich von 15 bis 100 mg, bezogen auf eine Applikationseinheit, enthält.

Als besonders vorteilhaft im Zusammenhang mit einer synergistischen Wirkverstärung von Schleimdroge einerseits und Polidocanol andererseits hat es sich erwiesen, wenn beide Substanzen in einem definierten gewichtsbezogenen Verhältnis zueinander in der Zusammensetzung vorliegen. Erfindungsgemäß enthält die Zusammensetzung (a) die mindestens eine Schleimdroge und/oder deren Extrakt einerseits und (b) Polidocanol andererseits in einem gewichtsbezogenen Verhältnis von [(a) : (b)] im Bereich von 1 : 5 bis 5 : 1.

Wie auch die erfindungsgemäßen Ausführungsbeispiele, auf welche noch im späteren Verlauf eingegangen wird, zeigen, lässt sich durch den Einsatz verschiedener gewichtsbezogener Verhältnisse eine differenziertere schmerzstillende Wirkung erzielen, als es bislang im Stand der Technik möglich ist.

Die Ausgestaltung der festen Dosierungsform ist variabel und wird im Folgenden zum besseren Verständnis detailliert erläutert:

Um eine effiziente Abgabe der eingesetzten Wirkstoffe an den Wirkort zu ermöglichen, kann es im Rahmen der vorliegenden Erfindung insbesondere vorgesehen sein, dass die Wirk- und/oder Inhaltsstoffe in eine feste Matrix und/oder Masse, vorzugsweise auf Basis von Zuckern und/oder Zuckeraustauschstoffen, eingelagert und/oder eingebettet sind.

In diesem Zusammenhang ist es besonders vorteilhaft, wenn die Zucker ausgewählt sind aus der Gruppe von Saccharose, Glucose, insbesondere Dextrose, und Fructose. Zudem kann es - insbesondere für die Herstellung zuckerfreier Ausführungsformen der erfindungsgemäßen Zusammensetzung - vorgesehen sein, dass die Zuckeraustauschstoffe aus Zuckeralkoholen und/oder aus der Gruppe von Mannit, Xylit, Sorbit, Isomalt, Maltitsirup, Lactit, Leucrose, Fructooligosacchariden, Glucanen, Polyglucose, besonders bevorzugt Isomalt, ausgewählt sind.

Die eingesetzte Menge an Zucker(n) bzw. Zuckeraustauschstoff(en) ist variabel. Üblicherweise enthält die erfindungsgemäße Zusammensetzung Zucker und/oder Zuckeraustauschstoff(e) in einer relativen Menge im Bereich von 65 bis 99,9 Gew.-%, insbesondere im Bereich von 80 bis 99 Gew.-%, vorzugsweise im Bereich von 85 bis 99 Gew.-%, bezogen auf die pharmazeutische Zusammensetzung.

Im Rahmen der vorliegenden Erfindung ist es vorgesehen, dass die feste Dosierungsform eine Tablette, Dragee, Pille, Hartkaramelle oder dergleichen, insbesondere eine Lutschtablette, ist.

Was die Funktionsweise der erfindungsgemäßen Zusammensetzung anbelangt, so beruht diese im Allgemeinen darauf, dass die feste Dosierungsform, insbesondere die Lutschtablette, derart gebildet ist, dass die feste Dosierungsform, insbesondere die Lutschtablette, eine therapeutisch wirksame Menge der Wirk- und/oder Inhaltsstoffe beim Lutschen freisetzt, wenn die feste Dosierungsform im Mund- und Rachenraum eines Patienten verabreicht und gelutscht wird.

Auch was die Größe der festen Dosierungsform anbelangt, so kann diese variieren. Vorzugsweise weist die pharmazeutische Zusammensetzung in Form einer Lutschtablette, insbesondere auf Hartkaramellenbasis, ein Gesamtgewicht im Bereich von 0,5 bis 5 g, insbesondere im Bereich von 0,8 bis 4 g, vorzugsweise im Bereich von 1 bis 3 g, auf.

Darüber hinaus kann es vorgesehen sein, dass die erfindungsgemäße Zusammensetzung mindestens einen weiteren Wirk- und/oder Inhaltsstoff enthält; der weitere Wirk- und/oder Inhaltstoff kann dabei insbesondere ausgewählt sein aus der Gruppe von Schleimhautschutzmitteln, Antiseptika, Vitaminen, Spurenelementen, Mineralien, Mikronährstoffen sowie deren Mischungen.

Darüber hinaus kann es ebenfalls vorgesehen sein, dass die Zusammensetzung außerdem mindestens einen weiteren Wirk- und/oder Inhaltsstoff enthält, welcher insbesondere ausgewählt sein kann aus der Gruppe von Verarbeitungshilfsmitteln, Färbe-, Puffer-, Riech-, Duft-, Streck-, Binde-, Netz- und/oder Konservierungsstoffen, pH-Stellmitteln, pH-Puffersubstanzen, Verdickungsmitteln, Aromastoffen, Geschmacksstoffen, Süßstoffen und Süßungsmitteln, Säuerungsmitteln, Stabilisatoren und/oder Antiseptika sowie deren Mischungen.

Gemäß einer besonders bevorzugten Ausführungsform ist Gegenstand der vorliegenden Erfindung eine Zusammensetzung in Form einer festen Dosierung, wie sie zuvor beschrieben worden ist, zur topischen Behandlung von entzündlichen Erkrankungen des Mund- und Rachenraumes, wobei die pharmazeutische Zusammensetzung in Kombination
(a) mindestens eine Schleimdroge und/oder deren Extrakt in einer relativen Menge im Bereich von 1 bis 10 Gew.-%;
(b) Polidocanol als Lokalanästhetikum in einer relativen Menge im Bereich von 0,1 bis 9 Gew.-%, vorzugsweise im Bereich von 0,5 bis 8 Gew.-%, bevorzugt im Bereich von 1 bis 7 Gew.-%, besonders bevorzugt im Bereich von 2 bis 5 Gew.-%;
(c) Zucker und/oder Zuckeraustauschstoffe in einer relativen Menge im Bereich von 65 bis 99 Gew.-%, insbesondere im Bereich von 80 bis 99 Gew.-%, vorzugsweise im Bereich von 85 bis 99 Gew.-%; aufweist, wobei die vorgenannten Mengenangaben jeweils auf die pharmazeutische Zusammensetzung bezogen sind.

Was die Herstellung der erfindungsgemäßen Zusammensetzung anbelangt, so kann diese mit üblichen, dem Fachmann an sich geläufigen Methoden erfolgen. Die Herstellung erfolgt insbesondere durch Mischen der einzelnen Wirk- bzw. Inhaltsstoffe, gefolgt von der nachfolgenden Verarbeitung bzw. Formgebung zu der gewünschten festen Dosierungsform, z. B. zu einer Lutschtablette. Dies ist dem Fachmann als solches geläufig, so dass es keiner weitergehenden diesbezüglichen Ausführungen bedarf.

In diesem Zusammenhang kann es insbesondere vorgesehen sein, die Zusammensetzung bei der topischen Behandlung von insbesondere schmerzbegleiteten entzündlichen Erkrankungen des Mund- und Rachenraumes zu verwenden.

Die vorliegende Erfindung wird anhand der folgenden Ausführungsbeispiele veranschaulicht, welche die vorliegende Erfindung jedoch keinesfalls beschränken.

### Ausführungsbeispiele:

### A) Herstellung erfindungsgemäßer Zusammensetzungen in Form von Lutschtabletten auf Hartkaramellenbasis und von Vergleichszusammensetzungen gleichermaßen in Form von Lutschtabletten auf Hartkaramellenbasis

Es wurden verschiedene erfindungsgemäße und nichterfindungsgemäße Zusammensetzungen in Form von Lutschtabletten auf Hartkaramellenbasis hergestellt, wobei als Zuckeraustauschstoff zur Einlagerung bzw. Einbettung der Wirk- und Inhaltsstoffe jeweils Isomalt (Palatinit^{®}) eingesetzt wurde. Gleichermaßen können aber auch Fructose- und/oder Glucosesirup und/oder andere Süßstoffe, wie beispielsweise Acesulfam, Aspartam, Cyclamat, Saccharin und dergleichen, als Matrixmaterial verwendet werden. Die Herstellung der Lutschtabletten bzw. Hartkaramellen erfolgte in dem Fachmann an sich bekannter Weise.

Die Ausgangsstoffe (Wirkstoffe, Geschmacksstoffe, Zusatz- bzw. Hilfsstoffe) wurden hierzu entsprechend der jeweiligen Rezeptur eingewogen und einzeln in das Matrixmaterial (Isomalt) eingemischt. Die Zugabe von Wasser war nicht notwendig. Der Vorgang fand unter Erwärmen statt. Die homogene Mischung aller Rohstoffe wurde in eine Dosiermaschine oder Dosiereinheit überführt. Dort wurden die Lutschtabletten in ihrer entsprechenden Größe hergestellt und während des Prozesses verwogen. Es resultierten Trockengewichte von 2,5 g pro Applikationseinheit bzw. pro Lutschtablette. Auf diese Weise wurden sowohl erfindungsgemäße Hartkaramellen als auch nichterfindungsgemäße Hartkaramellen zu Vergleichszwecken hergestellt.

Die erfindungsgemäßen Hartkaramellen A, B und C enthielten als Wirkstoffe jeweils 50 mg Isländisches Moos pro Applikationseinheit (Lutschtablette). Hinsichtlich der eingesetzten Menge an Polidocanol unterschieden sich die Hartkaramellen A, B und C voneinander: Hartkaramelle A enthielt 25 mg Polidocanol pro Applikationseinheit (1 Gew.-%, bezogen auf das Trockengewicht der Zusammensetzung); Hartkaramelle B enthielt 75 mg Polidocanol pro Applikationseinheit (3 Gew.-%, bezogen auf das Trockengewicht der Zusammensetzung); Hartkaramelle C enthielt pro Applikationseinheit 150 mg Polidocanol (6 Gew.-%, bezogen auf das Trockengewicht der Zusammensetzung). Die vollständigen Rezepturen der erfindungsgemäßen Hartkaramellen A, B und C sind der nachstehenden Tabelle 1 zu entnehmen.

**Tabelle 1: Rezepturen der erfindungsgemäßen Hartkaramellen A, B und C**

| **Rohstoffe** | **Menge (trocken) pro Lutschtablette A [mg]** | **Menge (trocken) pro Lutschtablette B [mg]** | **Menge (trocken) pro Lutschtablette C [mg]** |
|---|---|---|---|
| Palatinit^{®} (Isomalt) | 2.383,5 | 2.333,5 | 2.258,5 |
| Wasser | 0,0 | 0,0 | 0,0 |
| Isländisches Moos (Extrakt mit Droge/ Extrakt-Verhältnis 7:1) | 50,0 | 50,0 | 50,0 |
| Geschmacksstoffe | 26,5 | 26,5 | 26,5 |
| Polidocanol | 25,0 | 75,0 | 150,0 |
| Weinsäure | 15,0 | 15,0 | 15,0 |
| Gesamt | 2.500,0 | 2.500,0 | 2.500,0 |

Die nichterfindungsgemäßen Vergleichstabletten D und E unterschieden sich in Bezug auf die Wirkstoffe von den erfindungsgemäßen Vergleichstabletten: Die Vergleichstablette D enthielt als einzigen Wirkstoff Isländisches Moos in einer Menge von 50 mg pro Applikationseinheit. Die Vergleichstablette E enthielt als einzigen Wirkstoff 75 mg Polidocanol pro Applikationseinheit. Der nachstehenden Tabelle 2 sind die vollständigen Rezepturen der nichterfindungsgemäßen Vergleichslutschtabletten D und E zu entnehmen.

**Tabelle 2: Rezepturen der nichterfindungsgemäßen Hartkaramellen D und E**

| **Rohstoffe** | **Menge (trocken) pro Lutschtablette D [mg]** | **Menge (trocken) pro Lutschtablette E [mg]** |
|---|---|---|
| Palatinit^{®} (Isomalt) | 2.408,5 | 2.383,5 |
| Wasser | 0,0 | 0,0 |
| Isländisches Moos (Extrakt mit Droge/ Extrakt-Verhältnis 7: 1) | 50,0 | 0,0 |
| Geschmacksstoffe | 26,5 | 26,5 |
| Polidocanol | 0,0 | 75,0 |
| Weinsäure | 15,0 | 15,0 |
| Gesamt | 2.500,0 | 2.500,0 |

### B) Wirksamkeitsstudien mit den erfindungsgemäßen Lutschtabletten A, B und C sowie den Vergleichslutschtabletten D und E

Zur Untersuchung der Wirksamkeit der erfindungsgemäßen Lutschtabletten A, B und C sowie zum Vergleich mit den Vergleichslutschtabletten D und E wurde eine Probandengruppe mit insgesamt 75 Probanden im Alter von 20 bis 65 Jahren, von denen 37 weiblich und 38 männlich waren, herangezogen. Die Probanden litten an entzündlichen Erkrankungen im Hals-/Rachenraum und damit einhergehender Schmerzsymptomatik, wobei die Entzündungen auf Erkältungskrankheiten zurückzuführen waren.

Jeweils 15 Probanden erhielten dreimal täglich über einen Zeitraum von vier Tagen jeweils eine der Lutschtabletten A, B, C, D bzw. E. Im Rahmen der Behandlung wurde die jeweils getestete Zusammensetzung in Bezug auf die Linderung der Schmerzsymptomatik - einerseits im Zusammenhang mit der Nachhaltigkeit und andererseits mit der Anfangsintensität - sowie die entzündungshemmenden Effekte nach dem Schulnotensystem (1 = sehr gut bis 6 = ungenügend) bewertet. Die diesbezüglichen Ergebnisse sind der nachstehenden Tabelle 3 zu entnehmen.

**Tabelle 3: Bewertung der erfindungsgemäßen Zusammensetzungen sowie der Vergleichszusammensetzungen nach dem Schulnotensystem**

| **Zusammensetzung** | **Linderung Schmerzsymptomatik: Nachhaltigkeit** | **Linderung Schmerz-symptomatik: Anfangsintensität** | **Antiinflammatorische Wirkung** |
|---|---|---|---|
| A | | | |
| erfindungsgemäß 1 Gew.-% Polidocanol | 1,9 | 1,3 | 1,3 |
| B | | | |
| erfindungsgemäß 3 Gew.-% Polidocanol | 1,5 | 1,4 | 1,3 |
| C | | | |
| erfindungsgemäß 6 Gew.-% Polidocanol | 1,4 | 1,8 | 1,4 |
| D | | | |
| (Vergleich, Isländisches Moos Monopräparat) | 3,2 | 3,9 | 1,8 |
| E | | | |
| (Vergleich, Polidocanol Monopräparat) | 1,7 | 1,5 | 3,8 |

Bei allen drei erfindungsgemäßen Präparaten bzw. Lutschtabletten zeigte sich sowohl eine hervorragende entzündungshemmende Wirkung als auch eine effiziente Linderung der Schmerzsymptomatik. Was die antiinflammatorische Wirkung der erfindungsgemäßen Lutschtablette A, B und C anbelangt, waren keine Unterschiede zwischen den Präparaten zu erkennen. Alle erfindungsgemäßen Zusammensetzungen wurden im Zusammenhang mit der antiinflammatorischen Wirkung als sehr gut bewertet. Die unterschiedliche eingesetzte Menge an Polidocanol bzw. das unterschiedliche gewichtsbezogene Verhältnis von Polidocanol zu Isländischem Moos hingegen zeigte einen deutlichen Einfluss auf die schmerzlindernde Wirkung der Lutschtablette: So wurde die Anfangsintensität des lokalanästhetischen Effekts bei Einsatz einer Polidocanol-Menge von 1 Gew.-%, bezogen auf die Zusammensetzung, von den Patienten bzw. Probanden als sehr angenehm empfunden, da der Effekt nicht zu stark war; bedingt durch die geringere eingesetzte Menge war jedoch die Nachhaltigkeit der Linderung der Schmerzsymptomatik nicht immer optimal, wurde dennoch im Durchschnitt mit 1,9 bewertet und ist somit insgesamt als gut zu bewerten. Bei Einsatz von 6 Gew.-% Polidocanol (Lutschtablette C) wurde die starke Anfangsintensität der lokalanästhetischen Wirkung von den Probanden als unangenehmer empfunden als bei Einsatz von 1 Gew.-% Polidocanol; der Einsatz von 6 Gew.-% Polidocanol, bezogen auf die Zusammensetzung, führt jedoch zu einer hervorragenden Nachhaltigkeit in Bezug auf die Linderung der Schmerzsymptomatik. Das beste Ergebnis ließ sich mit der Zusammensetzung B erzielen, welche einen Polidocanol-Gehalt von 3 Gew.-% aufwies: Einerseits war die Anfangsintensität der lokalanästhetischen Wirkung nicht zu intensiv, dennoch konnte die Schmerzsymptomatik auch bei Einnahme von nur einer einzelnen Tablette äußerst nachhaltig bzw. über einen äußerst langen Zeitraum gelindert werden.

Mit den Vergleichszusammensetzungen D und E konnten keine zufriedenstellenden Ergebnisse erzielt werden. Die Vergleichszusammensetzung D, welche als einzigen Wirkstoff Isländisches Moos enthielt, ließ zumindest im Wesentlichen keine Linderung der Schmerzsymptomatik zu. Mit der Vergleichszusammensetzung E, bei welcher es sich um das Polidocanol-Monopräparat handelte, war zwar die schmerzstillende Wirkung zufriedenstellend, allerdings trat keine antiinflammatorische Wirkung auf. Bei keiner der getesteten Zusammensetzungen konnten beim Patienten Unverträglichkeitsreaktionen bzw. Schleimhautirritationen beobachten werden.

Die Untersuchungen zeigen, dass die Anwendung der erfindungsgemäßen Zusammensetzungen bei Erkrankung des Hals- und Rachenraumes einerseits zu einer effizienten Schmerzstillung führt, andererseits auch Entzündungen deutlich verringert werden können. Mit den Vergleichszusammensetzungen D und E konnte keine derartige Doppelwirkung erzeugt werden.

Darüber hinaus zeigen die erfindungsgemäßen Ausführungsbeispiele, dass zudem über die eingesetzte Polidocanol-Menge die Linderung der Schmerzsymptomatik gezielt gesteuert werden kann. Durch den Einsatz definierter Mengen kann einerseits verhindert werden, dass eine zu starke Anfangsintensität in Bezug auf den lokalanästhetischen Effekt auftritt, andererseits dennoch eine hervorragende Nachhaltigkeit bzw. lange andauernde Linderung der Schmerzsymptomatik gewährleistet wird. Hierin liegt ein entscheidender Vorteil der erfindungsgemäßen Kombination.

## Patentansprüche

1. Pharmazeutische Zusammensetzung in Form einer festen Dosierung zur Verwendung bei der topischen Behandlung von entzündlichen Erkrankungen des Mund- und Rachenraumes, wobei die feste Dosierungsform eine Tablette, Dragee, Pille oder Hartkaramelle ist und wobei die pharmazeutische Zusammensetzung in Kombination
(a) in einer relativen Menge im Bereich von 0,1 bis 25 Gew.-%, bezogen auf die pharmazeutische Zusammensetzung, mindestens eine Schleimdroge und/oder deren Extrakt; und
(b) in einer relativen Menge im Bereich von 0,1 bis 9 Gew.-%, bezogen auf die pharmazeutische Zusammensetzung, Polidocanol als Lokalanästhetikum;
aufweist,
wobei die Schleimdroge ausgewählt ist aus Isländischem Moos (*Lichen islandicus*), Eibisch (*Althaea officinalis L.*), Spitzwegerich (*Plantago lanceolata L.*), Malve (*Malva sylvestris L.* und *M*. *neglecta WALLR.*), Bockshornklee (*Trigonella foenum-graecum L.*), Salep und Quitte (*Cydonia oblonga MILL.*) und deren Kombinationen und
wobei die Zusammensetzung (a) die mindestens eine Schleimdroge und/oder deren Extrakt und (b) Polidocanol in einem gewichtsbezogenen Verhältnis von [(a) : (b)] im Bereich von 1 : 5 bis 5 : 1 enthält.

2. Zusammensetzung zur Verwendung nach Anspruch 1,
wobei die Zusammensetzung (a) die mindestens eine Schleimdroge in Form der Droge, insbesondere in Form zerkleinerter oder pulverisierter Pflanzenteile oder Pflanzenbestandteile, enthält; und/oder
wobei die Zusammensetzung (a) die mindestens eine Schleimdroge in Form eines Extraktes, insbesondere eines Trockenextraktes, enthält, insbesondere wobei der Extrakt ausgehend von einem wässrigen, alkoholischen oder wässrig-alkoholischen Extrakt, bevorzugt einem wässrigen Extrakt, erhältlich ist, insbesondere wobei die Zusammensetzung (a) die mindestens eine Schleimdroge in Form eines Extraktes, vorzugsweise eines Trockenextraktes, mit einem Droge/Extrakt-Verhältnis im Bereich von 0,2 : 1 bis 20 : 1, insbesondere im Bereich von 1 : 1 bis 15 : 1, vorzugsweise im Bereich von 5: 1 bis 10 : 1, enthält.

3. Zusammensetzung zur Verwendung nach Anspruch 1 oder 2,
wobei die Zusammensetzung (a) die mindestens eine Schleimdroge und/oder deren Extrakt in einer relativen Menge im Bereich von 1 bis 10 Gew.-%, bezogen auf die pharmazeutische Zusammensetzung, enthält; und/oder
wobei die Zusammensetzung (a) die mindestens eine Schleimdroge und/oder deren Extrakt in einer absoluten Menge im Bereich von 1 bis 500 mg, insbesondere im Bereich von 10 bis 250 mg, vorzugsweise im Bereich von 20 bis 200 mg, bevorzugt im Bereich von 30 bis 100 mg, bezogen auf eine Applikationseinheit, enthält.

4. Zusammensetzung zur Verwendung nach einem der vorangehenden Ansprüche,
wobei die mindestens eine Schleimdroge ausgewählt ist aus Isländischem Moos (*Lichen islandicus*) und/oder Eibisch (*Althaea officinalis L.*), vorzugsweise aus Isländischem Moos (*Lichen islandicus*); und/oder
wobei die Schleimdroge Isländisches Moos (*Lichen islandicus L.*) und/oder Eibisch (*Althaea officinalis L.*)*,* insbesondere Isländisches Moos (*Lichen islandicus L.*), ist.

5. Zusammensetzung zur Verwendung nach einem der vorangehenden Ansprüche,
wobei die Zusammensetzung (b) das Polidocanol in einer relativen Menge im Bereich von 1 bis 7 Gew.-%, besonders bevorzugt im Bereich von 2 bis 5 Gew.-%, bezogen auf die pharmazeutische Zusammensetzung, enthält; und/oder
wobei die Zusammensetzung (b) das Polidocanol in einer absoluten Menge im Bereich von 1 bis 500 mg, insbesondere im Bereich von 2 bis 250 mg, vorzugsweise im Bereich von 5 bis 200 mg, bevorzugt im Bereich von 10 bis 150 mg, besonders bevorzugt im Bereich von 15 bis 100 mg, bezogen auf eine Applikationseinheit, enthält.

6. Zusammensetzung zur Verwendung nach einem der vorangehenden Ansprüche,
wobei die Wirk- und/oder Inhaltsstoffe in eine feste Matrix und/oder Masse auf Basis von Zuckern und/oder Zuckeraustauschstoffen eingelagert und/oder eingebettet sind;
insbesondere wobei die Zucker ausgewählt sind aus der Gruppe von Saccharose, Glucose, insbesondere Dextrose, und Fructose und/oder wobei die Zuckeraustauschstoffe ausgewählt sind aus Zuckeralkoholen und/oder aus der Gruppe von Mannit, Xylit, Sorbit, Isomalt, Maltitsirup, Lactit, Leucrose, Fructooligosacchariden, Glucanen, Polyglucose, besonders bevorzugt Isomalt; und/oder
insbesondere wobei die Zusammensetzung Zucker und/oder Zuckeraustauschstoffe in einer relativen Menge im Bereich von 65 bis 99,9 Gew.-%, insbesondere im Bereich von 80 bis 99 Gew.-%, vorzugsweise im Bereich von 85 bis 99 Gew.-%, bezogen auf die pharmazeutische Zusammensetzung, enthält.

7. Zusammensetzung zur Verwendung nach einem der vorangehenden Ansprüche,
wobei die feste Dosierungsform eine Tablette, Dragee, Pille, Hartkaramelle oder dergleichen, insbesondere eine Lutschtablette, ist; und/oder
wobei die feste Dosierungsform, insbesondere die Lutschtablette, derart gebildet ist, dass die feste Dosierungsform, insbesondere die Lutschtablette, eine therapeutisch wirksame Menge der Wirk- und/oder Inhaltsstoffe beim Lutschen freisetzt, wenn die feste Dosierungsform im Mund - und Rachenraum eines Patienten verabreicht und gelutscht wird; und/oder
wobei die pharmazeutische Zusammensetzung in Form einer Lutschtablette, insbesondere auf Hartkaramellenbasis, vorliegt, wobei die Lutschtablette ein Gesamtgewicht im Bereich von 0,5 bis 5 g, insbesondere im Bereich von 0,8 bis 4 g, vorzugsweise im Bereich von 1 bis 3 g, aufweist.

8. Zusammensetzung zur Verwendung nach einem der vorangehenden Ansprüche,
wobei die Zusammensetzung mindestens einen weiteren Wirk- und/oder Inhaltsstoff, insbesondere ausgewählt aus der Gruppe von Schleimhautschutzmitteln, Antiseptika, Vitaminen, Spurenelementen, Mineralien, Mikronährstoffen sowie deren Mischungen, enthält; und/oder
wobei die Zusammensetzung außerdem mindestens einen weiteren Wirk- und/oder Inhaltsstoff, insbesondere ausgewählt aus der Gruppe von Verarbeitungshilfsmitteln, Färbe-, Puffer-, Riech-, Duft-, Streck-, Binde-, Netz- und/oder Konservierungsstoffen, pH-Stellmitteln, pH-Puffersubstanzen, Verdickungsmitteln, Aromastoffen, Geschmacksstoffen, Süßstoffen und Süßungsmitteln, Säuerungsmitteln, Stabilisatoren und/oder Antiseptika sowie deren Mischungen, enthält.

9. Zusammensetzung in Form einer festen Dosierung nach einem der vorangehenden Ansprüche zur Verwendung bei der topischen Behandlung von entzündlichen Erkrankungen des Mund- und Rachenraumes, wobei die feste Dosierungsform eine Tablette, Dragee, Pille oder Hartkaramelle ist und wobei die pharmazeutische Zusammensetzung in Kombination
(a) mindestens eine Schleimdroge und/oder deren Extrakt in einer relativen Menge im Bereich von 0,1 bis 25 Gew.-%, insbesondere im Bereich von 1 bis 10 Gew.-%;
(b) Polidocanol als Lokalanästhetikum in einer relativen Menge im Bereich von 0,1 bis 9 Gew.-%, vorzugsweise im Bereich von 0,5 bis 8 Gew.-%, bevorzugt im Bereich von 1 bis 7 Gew.-%, besonders bevorzugt im Bereich von 2 bis 5 Gew.-%;
(c) Zucker und/oder Zuckeraustauschstoffe in einer relativen Menge im Bereich von 65 bis 99 Gew.-%, insbesondere im Bereich von 80 bis 99 Gew.-%, vorzugsweise im Bereich von 85 bis 99 Gew.-%;
aufweist, wobei die vorgenannten Mengenangaben jeweils auf die pharmazeutische Zusammensetzung bezogen sind,
wobei die Schleimdroge ausgewählt ist aus Isländischem Moos (*Lichen islandicus*), Eibisch (*Althaea officinalis L.*), Spitzwegerich (*Plantago lanceolata L.*), Malve (*Malva sylvestris L.* und *M. neglecta WALLR.*), Bockshornklee (*Trigonella foenum-graecum L.*), Salep und Quitte (*Cydonia oblonga MILL.*) und deren Kombinationen und
wobei die Zusammensetzung (a) die mindestens eine Schleimdroge und/oder deren Extrakt und (b) Polidocanol in einem gewichtsbezogenen Verhältnis von [(a) : (b)] im Bereich von 1 : 5 bis 5 : 1 enthält.

10. Verwendung einer Zusammensetzung, wie in einem der vorangehenden Ansprüche definiert, zur Herstellung eines Arzneimittels zur prophylaktischen und/oder therapeutischen Behandlung des menschlichen oder tierischen Körpers.

11. Verwendung einer pharmazeutischen Zusammensetzung, wie in einem der vorangehenden Ansprüche definiert, zur Herstellung eines Arzneimittels zur topischen Behandlung von insbesondere schmerzbegleiteten entzündlichen Erkrankungen des Mund- und Rachenraums.

## Claims

1. Pharmaceutical composition in the form of a fixed dosage for use in the topical treatment of inflammatory diseases of the mouth and throat, wherein the solid dosage form is a tablet, dragee, pill or hard caramel and wherein the pharmaceutical composition in combination, has
(a) at least one mucilaginous drug and/or its extract in a relative amount in the range of 0.1 to 25 wt .-%, based on the pharmaceutical composition; and
(b) polidocanol as a local anaesthetic in a relative amount in the range of 0.1 to 9 wt .-%, based on the pharmaceutical composition
wherein the mucilaginous drug is selected from Icelandic moss (*Lichen islandicus*), marshmallow (*Althaea officinalis L.*), plantain (*Plantago lanceolata L.*), mallow (*Malva sylvestris L.* and *M. neglecta WALLR.*), fenugreek (*Trigonella foenum-graecum L.*), salep and quince (*Cydonia oblonga MILL.*) and combinations thereof, and
wherein the composition contains (a) the at least one mucilaginous drug and/or its extract and (b) polidocanol in a weight ratio [(a):(b)] in the range from 1:5 to 5:1.

2. Composition for use according to claim 1,
wherein the composition contains (a) the at least one mucilaginous drug in the form of the drug, particularly in the form of comminuted or pulverised plant parts or plant constituents; and/or
wherein the composition contains (a) the at least one mucilaginous drug in the form of an extract, in particular a dry extract, in particular wherein the extract is obtained from an aqueous, alcoholic or aqueous-alcoholic extract, preferably an aqueous extract, in particular wherein the composition contains (a) the at least one mucilaginous drug in the form of an extract, preferably a dry extract with a drug/extract ratio in the range from 0.2:1 to 20:1, in particular in the range from 1:1 to 15:1, preferably in the range from 5:1 to 10:1.

3. Composition for use according to claim 1 or 2,
wherein the composition contains (a) the at least one mucilaginous drug and/or its extract in a relative amount in the range from 1 to 10 wt .-%, based on the pharmaceutical composition; and/or
wherein the composition contains (a) the at least one mucilaginous drug and/or its extract in an absolute amount in the range from 1 to 500 mg, in particular in the range from 10 to 250 mg, preferably in the range from 20 to 200 mg, more preferably in the range from 30 to 100 mg, based on an application unit.

4. Composition for use according to any one of the preceding claims,
wherein the at least one mucilaginous drug is selected from Icelandic moss (*Lichen islandicus*) and/or marshmallow (*Althaea officinalis L.*), preferably from Icelandic moss (*Lichen islandicus*); and/or
where the mucilaginous drug is Icelandic moss (*Lichen islandicus L.*) and/or marshmallow (*Althaea officinalis L.*), in particular Icelandic moss (*Lichen islandicus L.*).

5. Composition for use according to any one of the preceding claims,
wherein the composition contains (b) the polidocanol in a relative amount in the range from 1 to 7 wt .-%, particularly preferably in the range from 2 to 5 wt .-%, based on the pharmaceutical composition; and/or
wherein the composition contains (b) the polidocanol in an absolute amount in the range from 1 to 500 mg, in particular in the range from 2 to 250 mg, preferably in the range from 5 to 200 mg, more preferably in the range from 10 to 150 mg, particularly preferably in the range of 15 to 100 mg, based on an application unit.

6. Composition for use according to any one of the preceding claims,
wherein the active ingredients and/or substances are incorporated and/or embedded into a solid matrix and/or mass based on sugars and/or sugar substitutes;
in particular wherein the sugars are selected from the group consisting of sucrose, glucose, in particular dextrose and fructose and/or where the sugar substitutes are selected from sugar alcohols and/or from the group of mannitol, xylitol, sorbitol, isomalt, maltitol syrup, lactitol, leucrose, fructooligosaccharides, glucans, polyglucose, particularly preferably isomalt; and/or
in particular wherein the composition contains sugar and/or sugar substitutes, in a relative amount in the range from 65 to 99.9 wt .-%, in particular in the range from 80 to 99 wt .-%, preferably in the range from 85 to 99 wt .-% based on the pharmaceutical composition.

7. Composition for use according to any one of the preceding claims,
wherein the solid dosage form is a tablet, dragee, pill, hard caramel or the like, in particular a lozenge; and/or
wherein the solid dosage form, in particular the lozenge, is so formed that the solid dosage form, in particular the lozenge, liberates a therapeutically-effective amount of the active ingredients and/or substances during sucking when the solid dosage form is administered in the mouth and throat of a patient and is sucked; and/or
wherein the pharmaceutical composition is present in the form of a lozenge, in particular with a hard caramel base, wherein the lozenge has a total weight in the range from 0.5 to 5 g, particularly in the range from 0.8 to 4 g, preferably in the range from 1 to 3 g.

8. Composition for use according to any one of the preceding claims,
wherein the composition contains at least one further active substance and/or ingredient, in particular selected from the group of mucosal protective agents, antiseptics, vitamins, trace elements, minerals, micronutrients and mixtures thereof; and/or
wherein the composition further contains at least one further active substance and/or ingredient, in particular selected from the group of processing aids, colorants, buffers, fragrances, perfumes, extenders, binders, wetting agents and/or preservatives, pH-suspending agents, pH-buffer substances, thickening agents, flavouring agents, flavours, sweeteners and sweetening agents, acidifiers, stabilizers and/or antiseptics, and mixtures thereof.

9. Composition in the form of a fixed dosage according to any one of the preceding claims for use in the topical treatment of inflammatory diseases of the mouth and throat, wherein the solid dosage form is a tablet, dragee, pill or hard caramel and wherein the pharmaceutical composition in combination, has
(a) at least a mucilaginous drug and/or its extract in a relative amount in the range from 0.1 to 25 wt .-%, in particular in the range from 1 to 10 wt .-%;
(b) polidocanol as a local anaesthetic in a relative amount in the range from 0.1 to 9 wt.-%, preferably in the range from 0.5 to 8 wt .-%, more preferably in the range from 1 to 7 wt .-%, particularly preferably in the range from 2 to 5 wt .-%;
(c) sugar and/or sugar substitutes, in a relative amount in the range from 65 to 99 wt.-%, in particular in the range from 80 to 99 wt.-%, preferably in the range from 85 to 99 wt .-%;
wherein the above-mentioned amounts are in each case based on the pharmaceutical composition,
wherein the mucilaginous drug is selected from Icelandic moss (*Lichen islandicus*), marshmallow (*Althaea officinalis L.*)*,* plantain (*Plantago lanceolata L.*)*,* mallow (*Malva sylvestris L.* and *M. neglecta WALLR.*), fenugreek (*Trigonella foenum-graecum L* .), salep and quince (*Cydonia oblonga MILL.*) and combinations thereof, and
wherein the composition contains (a) the at least one mucilaginous drug and/or its extract and (b) polidocanol in a weight ratio of [(a): (b)] in the range from 1:5 to 5:1.

10. Use of a composition according to any one of the preceding claims, for the production of a medicament for the prophylactic and/or therapeutic treatment of the human or animal body.

11. Use of a composition according to any one of the preceding claims for the production of a medicament for the topical treatment in particular of pain-related inflammatory diseases of the mouth and throat.

## Revendications

1. Composition pharmaceutique sous forme d'une posologie solide pour utilisation lors du traitement topique de maladies inflammatoires de l'espace oropharyngé, la forme posologique solide étant un comprimé, une dragée, une pilule ou un caramel dur, et la composition pharmaceutique comportant, en association
(a) selon une quantité relative comprise dans la plage de 0,1 à 25 % en poids par rapport à la composition pharmaceutique, d'au moins une plante médicinale mucilagineuse et/ou d'un extrait de cette dernière ; et
(b) selon une quantité relative comprise dans la plage de 0,1 à 9 % en poids par rapport à la composition pharmaceutique, de polidocanol en tant qu'anesthésique local ;
la plante médicinale mucilagineuse étant choisie parmi la mousse d'Islande (*Lichen islandicus*)*,* la guimauve officinale (*Althaea officinalis L.*), le plantain lancéolé (*Plantago lanceolata L.*), la mauve (*Malva sylvestris L.* et *M. neglecta WALLR*.), le fénugrec (*Trigonella foenum-graecum L.*), le salep et le cognassier (*Cydonia oblonga MILL.*) et leurs combinaisons, et
la composition contenant (a) la ou les plantes médicinales mucilagineuses et/ou l'extrait de ces dernières et (b) le polidocanol selon un rapport en poids [(a) : (b)] compris dans la plage de 1:5 à 5:1.

2. Composition pour l'utilisation selon la revendication 1,
la composition contenant (a) la ou les plantes médicinales mucilagineuses sous forme de la plante médicinale, en particulier sous forme de parties de plantes ou de constituants de plantes broyés ou pulvérisés ; et/ou
la composition contenant (a) la ou les plantes médicinales mucilagineuses sous forme d'un extrait, en particulier d'un extrait sec, en particulier l'extrait pouvant être obtenu à partir d'un extrait aqueux, alcoolique ou hydro-alcoolique, de préférence d'un extrait aqueux, en particulier la composition contenant (a) la ou les plantes médicinales mucilagineuses sous forme d'un extrait, de préférence d'un extrait sec, avec un rapport plante médicinale/extrait compris dans la plage de 0,2:1 à 20:1, en particulier dans la plage de 1:1 à 15:1, de préférence dans la plage de 5:1 à 10:1.

3. Composition pour l'utilisation selon la revendication 1 ou 2,
la composition contenant (a) la ou les plantes médicinales mucilagineuses et/ou l'extrait de ces dernières selon une quantité relative comprise dans la plage de 1 à 10 % en poids par rapport à la composition pharmaceutique ; et/ou
la composition contenant (a) la ou les plantes médicinales mucilagineuses et/ou l'extrait de ces dernières selon une quantité absolue comprise dans la plage de 1 à 500 mg, en particulier dans la plage de 10 à 250 mg, de préférence dans la plage de 20 à 200 mg, de préférence dans la plage de 30 à 100 mg, par rapport à une forme d'administration unitaire.

4. Composition pour l'utilisation selon l'une des revendications précédentes,
la ou les plantes médicinales mucilagineuses étant choisies parmi la mousse d'Islande (*Lichen islandicus*) et/ou la guimauve officinale (*Althaea officinalis L.*), de préférence parmi la mousse (*Lichen islandicus*) ; et/ou
la plante médicinale mucilagineuse étant la mousse d'Islande (*Lichen islandicus L.*) et/ou la guimauve officinale (*Althaea officinalis L.*), en particulier la mousse d'Islande (*Lichen islandicus L.*).

5. Composition pour l'utilisation selon l'une des revendications précédentes,
la composition contenant (b) le polidocanol selon une quantité relative comprise dans la plage de 1 à 7 % en poids, d'une manière particulièrement préférée dans la plage de 2 à 5 % en poids, par rapport à la composition pharmaceutique ; et/ou
la composition contenant (b) le polidocanol selon une quantité absolue comprise dans la plage de 1 à 500 mg, en particulier dans la plage de 2 à 250 mg, de préférence dans la plage de 5 à 200 mg, préférentiellement dans la plage de 10 à 150 mg, d'une manière particulièrement préférée dans la plage de 15 à 100 mg, par rapport à une forme d'administration unitaire.

6. Composition pour l'utilisation selon l'une des revendications précédentes,
dans laquelle les principes actifs et/ou constituants sont incorporés et/ou insérés dans une matrice solide et/ou dans une masse à base de sucres et/ou de succédanés du sucre ;
en particulier dans laquelle les sucres sont choisis dans le groupe consistant en le saccharose, le glucose, en particulier le dextrose, et le fructose, et/ou dans laquelle les succédanés du sucre sont choisis parmi les alcools de sucre et/ou dans le groupe consistant en le mannitol, le xylitol, le sorbitol, l'isomaltol, le sirop de maltitol, le lactitol, le leucrose, les fructo-oligosaccharides, les glucanes, le polyglucose, d'une manière particulièrement préférée l'isomaltol ; et/ou
en particulier la composition contenant les sucres et/ou les succédanés du sucre selon une quantité relative comprise dans la plage de 65 à 99,9 % en poids, en particulier dans la plage de 80 à 99 % en poids, de préférence dans la plage de 85 à 99 % en poids, par rapport à la composition pharmaceutique.

7. Composition pour l'utilisation selon l'une des revendications précédentes,
la forme posologique solide étant un comprimé, une dragée, une pilule, un caramel dur ou analogues, en particulier un comprimé à sucer ; et/ou
la forme posologique solide, en particulier le comprimé à sucer, étant conçu de telle sorte que la forme posologique solide, en particulier le comprimé à sucer, libère une quantité thérapeutiquement efficace des principes actifs et/ou des constituants lors du suçage, quand la forme posologique solide est administrée dans l'espace oropharyngé de patients, et est sucée ; et/ou
la composition pharmaceutique se présentant sous la forme d'un comprimé à sucer, en particulier à base de caramel dur, le comprimé à sucer présentant un poids total compris dans la plage de 0,5 à 5 g, en particulier dans la plage de 0,8 à 4 g, de préférence dans la plage de 1 à 3 g.

8. Composition pour l'utilisation selon l'une des revendications précédentes,
la composition contenant au moins un principe actif et/ou un constituant supplémentaire, choisi en particulier dans le groupe consistant en les agents protecteurs de la muqueuse, les antiseptiques, les vitamines, les oligo-éléments, les substances minérales, les micronutriments, ainsi que les mélanges de ceux-ci ; et/ou
la composition contenant par ailleurs au moins un principe actif et/ou un constituant supplémentaire, choisi en particulier dans le groupe consistant en les auxiliaires de mise en oeuvre, les colorants, les substances tampon, les substances odorantes, les parfums, les diluants, les liants, les mouillants et/ou les conservateurs, les agents d'ajustement du pH, les substances tampon de pH, les épaississants, les aromatisants, les agents de sapidité, les édulcorants et les agents édulcorants, les acidifiants, les stabilisants et/ou les antiseptiques, ainsi que les mélanges de ceux-ci.

9. Composition sous la forme d'une posologie solide selon l'une des revendications précédentes, pour une utilisation lors du traitement topique de maladies inflammatoires de l'espace oropharyngé, la forme posologique solide étant un comprimé, une dragée, une pilule ou un caramel dur, et la composition pharmaceutique comprenant, en association,
(a) au moins une plante médicinale mucilagineuse et/ou un extrait de cette dernière selon une quantité relative comprise dans la plage de 0,1 à 25 % en poids, en particulier dans la plage de 1 à 10 % en poids ;
(b) du polidocanol en tant qu'anesthésique local selon une quantité relative comprise dans la plage de 0,1 à 9 % en poids, de préférence dans la plage de 0,5 à 8 % en poids, préférentiellement dans la plage de 1 à 7 % en poids, d'une manière particulièrement préférée dans la plage de 2 à 5 % en poids ;
(c) des sucres et/ou des succédanés du sucre selon une quantité relative comprise dans la plage de 65 à 99 % en poids, en particulier dans la plage de 80 à 99 % en poids, de préférence dans la plage de 85 à 99 % en poids ;
les indications de quantité ci-dessus étant chacune rapportées à la composition pharmaceutique
la plante médicinale mucilagineuse étant choisie parmi la mousse d'Islande (*Lichen islandicus*), la guimauve officinale (*Althaea officinalis L.*), le plantain lancéolé (*Plantago lanceolata L.*), la mauve (*Malva sylvestris L.* et *M. neglecta WALLR.*), le fénugrec (*Trigonella foenum-graecum L.*), le salep et le cognassier (*Cydonia oblonga MILL.*) et leurs combinaisons, et
la composition contenant (a) la ou les plantes médicinales mucilagineuses et/ou l'extrait de ces dernières et (b) le polidocanol selon un rapport en poids [(a) : (b)] compris dans la plage de 1:5 à 5:1.

10. Utilisation d'une composition telle que définie dans l'une des revendications précédentes pour la fabrication d'un médicament destiné au traitement prophylactique et/ou thérapeutique du corps humain ou animal.

11. Utilisation d'une composition pharmaceutique telle que définie dans l'une des revendications précédentes, pour la fabrication d'un médicament destiné au traitement topique de maladies inflammatoires, en particulier s'accompagnant de douleurs, de l'espace oropharyngé.
